(19) Europäisches Patentamt
European Patent Office
Office européen des brevets

(11) Veröffentlichungsnummer: **0 432 454 B1**

(12) **EUROPÄISCHE PATENTSCHRIFT**

(45) Veröffentlichungstag der Patentschrift: **07.09.94**

(51) Int. Cl.5: **C12N 9/22**, C12N 1/20

(21) Anmeldenummer: **90121351.2**

(22) Anmeldetag: **08.11.90**

(54) **Typ II-Restriktionsendonuklease SgrAI.**

(30) Priorität: **16.11.89 DE 3938143**

(43) Veröffentlichungstag der Anmeldung:
**19.06.91 Patentblatt 91/25**

(45) Bekanntmachung des Hinweises auf die Patenterteilung:
**07.09.94 Patentblatt 94/36**

(84) Benannte Vertragsstaaten:
**AT BE CH DE DK ES FR GB GR IT LI LU NL SE**

(56) Entgegenhaltungen:

NUCLEIC ACIDS RESEARCH. vol. 18, no. 10, 25 Mai 1990, ARLINGTON, VIRGINIA Seite 3087 TAUTZ, N. et al.: "SgrAI, a novel class-II restriction endonuclease from Streptomyces griseus recognizing the octanucleotide sequence 5'-CR/CCGGYG-3'"

(73) Patentinhaber: **BOEHRINGER MANNHEIM GMBH**

**D-68298 Mannheim (DE)**

(72) Erfinder: **Kaluza, Klaus, Dr.rer.nat.**
**Hochfeldanger 3**
**W-8173 Bad Heilbrunn (DE)**
Erfinder: **Frey, Bruno, Dr.rer.nat.**
**Hochfeldstrasse 50**
**W-8122 Penzberg (DE)**
Erfinder: **Schmitz-Agheguian, Gudrun., Dr.rer.nat.**
**Wettersteinstrasse 3**
**W-8139 Bernried (DE)**
Erfinder: **Jarsch, Michael, Dr.rer.nat.**
**Unterkarpfsee 11**
**W-8173 Bad Heilbrunn (DE)**
Erfinder: **Kessler, Christoph, Dr.rer.nat.**
**Schlossbergweg 11**
**W-8021 Dorfen (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services
(3.10/3.09/3.3.3)

**Beschreibung**

Die Erfindung betrifft die neue Typ II-Restriktionsendonuklease SgrAI, ein Verfahren zu ihrer Gewinnung und ihre Verwendung.

Typ II-Restriktionsendonukleasen sind Endodesoxyribonukleasen, welche bestimmte DNA-Sequenzen zu erkennen und zu spalten vermögen. Dabei werden Phosphodiesterbrücken in der Zielsequenz hydrolysiert und zwar in jedem Polynukleotidstrang eine. Typ II-Restriktionsendonukleasen sind daher wertvoll für die Analyse von DNA-Molekülen. Es sind zwar bereits für zahlreiche DNA-Sequenzen spezifische Typ II-Restriktionsendonukleasen bekannt, jedoch besteht immer noch Bedarf an der Schaffung weiterer Typ II-Restriktionsendonukleasen, die für DNA-Sequenzen spezifisch sind und die bisher von keiner der bekannten Restriktionsendonukleasen erkannt werden. Der Erfindung liegt daher die Aufgabe zu Grunde, eine neue Restriktionsendonuklease zur Verfügung zu stellen, welche eine bisher von keinem derartigen Enzym erkannte Sequenz spezifisch zu erkennen und zu spalten vermag.

Gelöst wird diese Aufgabe erfindungsgemäß durch eine Typ-II-Restriktionsendonuklease mit der Erkennungssequenz und der durch die Markierung definierten Spaltstelle

$$5'\text{-C}\genfrac{}{}{0pt}{}{A}{G}\,|\,\text{CCGG}\genfrac{}{}{0pt}{}{C}{T}\text{G-3'}$$

$$3'\text{-G}\genfrac{}{}{0pt}{}{C}{T}\,\text{GGCC}\,|\,\genfrac{}{}{0pt}{}{A}{G}\text{C-5'},$$

welche aus Mikroorganismen der Gattung Streptomyces erhältlich ist.

Die erfindungsgemäße neue Restriktionsendonuklease, die im folgenden als SgrAI bezeichnet wird, hat ein Temperaturoptimum bei 37°C. Das Enzym besitzt eine gute Aktivität zwischen pH 7,5 und pH 8,5 in 33 mmol/1 Tris-Acetat-Puffer mit 1,0 mmol/1 DTE (Dithioerythritol) 10 mmol/1 $MgCl_2$ und 66 mmol/1 K-Acetat. Das pH-Optimum liegt bei ca. pH 7,9. Ein Enzym, das isoschizomer zu SgrAI ist, ist nicht bekannt.

Die Erkennungssequenz läßt sich durch vollständigen Verdau der DNAs der Viren SV40 und Adeno 2, der Phagen Lambda, phiX1174 und dem Phagenderivaten M13mp8 und den Plasmiden pBR322 und pBR328 bestätigen. Diese DNA-Moleküle werden mit SgrAI behandelt.

Tabelle I zeigt einen Vergleich der experimentell beobachteten Schnittstellenspezifität mit einer computerermittelten Schnittstellenspezifität für ein Enzym, welches folgende Sequenz erkennt:

$$5'\text{-C}\genfrac{}{}{0pt}{}{A}{G}\text{CCGG}\genfrac{}{}{0pt}{}{C}{T}\text{G-3'}$$

Tabelle I

| DNA | Anzahl der experimentell bestimmten Schnittstellen | Anzahl der durch Computeranalyse bestimmte Schnittstellen | Experimentell bestimmte Fragmentlängen (Basenpaare) | Durch Computeranalyse bestimmte Fragmentlängen (Basenpaare) | Spaltpositionen ermittelt durch Computeranalyse (bei Basenpaar) |
|---|---|---|---|---|---|
| SV40 | 0 | 0 | 0 | 0 | 0 |
| M13mp8 | 0 | 0 | 0 | 0 | 0 |
| phiX174 | 0 | 0 | 0 | 0 | 0 |
| pBR322 | 1 | 1 | 4400 | 4363 | 408 |
| pBR328 | 1 | 1 | 4900 | 4907 | 409 |
| Lambda | 6 | 6 | 17000 15000 7000 4200 2800 1600 1300 | 16679 14850 7063 4198 2775 1616 1321 | 7064 8680 12878 15653 16974 31824 |
| Ad | 6 | 6 | 17000 9200 6000 2700 630 330 180 | 16978 9175 5915 2727 625 334 184 | 184 808 17786 23701 26428 26762 |

Die Spaltposition innerhalb der Erkennungssequenz des Enzyms läßt sich an einem M13-Derivat, das im Abstand von ca. 30 - 200 Basen zur Bindungsstelle des universalen Sequenzierprimers (Messing, J. et al. (1981) Nucl. Acids Res. 9, 309 - 321) diese Erkennungssequenz trägt, bestimmen. An einzelsträngiger DNS des M13-Derivates werden zunächst mit dem universalen Sequenzierprimer Sequenzreaktionen nach der Didesoxy-Kettenabbruchmethode durchgeführt (Sanger, F., et al. (1977) Proc. Natl. Acad. Sci. USA 74, 560 - 564, Messing, J. et al. (1981) Nucl. Acids Res. 9, 309 - 321).

Parallel dazu wird der Sequenzierprimer mit T4-Polynukleotid-Kinase und [$\gamma$-$^{32}$P]ATP am 5′-Ende radioaktiv markiert. Nach Anhybridisieren dieses 5′-endmarkierten Sequenzierprimers an die einzelsträngige M13-DNS wird in einer Auffüllreaktion mit DNS Polymerase I, (Klenow Enzym) und einer Desoxynukleotidtriphosphatmischung aus dATP, dCTP, dGTP und dTTP eine partiell doppelsträngige DNS hergestellt. Diese DNS, deren neusynthetisierter Strang am 5′-Ende radioaktiv markiert ist, wird mit der Restriktionsendonuklease SgrAI gespalten. Die Hälfte des Spaltansatzes wird zusätzlich noch mit T4 DNS Polymerase in Gegenwart einer Mischung aller vier Desoxynukleotidtriphosphate behandelt, um glatte DNS-Enden zu erhalten.

Die Analyse der Reaktionsprodukte erfolgt durch Elektrophorese auf Sequenziergelen (8 mol/l Harnstoff, 5 % Polyacrylamid) und anschließender Autoradiographie. Die Interpretation der Ergebnisse wird nach Brown, N.L. und Smith, M. (Methods in Enzymology 65 (1980) 391 - 401) durchgeführt. Durch Vergleich der Laufstrecken der radioaktiv markierten Fragmente mit der Sequenzleiter wird die Lage der Spaltstelle bestimmt. Die zusätzlich mit T4 DNS Polymerase behandelten Proben zeigen im Vergleich zu der lediglich mit SgrAI gespaltenen Probe eine um 4bp verkürzte Laufstrecke der Banden. Damit ist gezeigt, daß SgrAI ein um 4bp 5′-überhängendes DNS-Ende erzeugt. Die Spaltung von SgrAI erfolgt innerhalb der Erkennungssequenz daher mit folgender Spezifität:

$$5'-C{\,}^A_G \Big| CCGG{\,}^C_T G-3'$$

$$3'-G{\,}^C_T \; GGCC \Big| {\,}^A_G C-5'$$

Die experimentell bestimmte Anzahl der Schnittstellen ist identisch mit der Anzahl der Schnittstellen, die durch Computeranalyse mit den verschiedenen DNAs für die Sequenz

$$5'-C{\,}^A_G CCGG{\,}^C_T G-3'$$

erhalten wurde (Tabelle I). Zusätzlich wurden diese Daten noch mit den Tabellen aus Gene 10 (1980) 357 - 370, verglichen.

Vorzugsweise wird SgrAI gewonnen, indem man Mikroorganismen der Gattung Streptomyces züchtet und das Enzym aus den Zellen gewinnt. Vorzugsweise wird Streptomyces griseus • DSM 5621 verwendet.

Der Mikroorganismus Streptomyces griseus ist bei der Deutschen Sammlung von Mikroorganismen (DSM), Mascheroder Weg 1 b, 3300 Braunschweig, BRD, hinterlegt und trägt die Hinterlegungsnummer DSM 5621.

Zur Gewinnung können die üblichen biochemischen Aufreinigungsmethoden verwendet werden, wobei in den jeweils erhaltenen Fraktionen das Vorhandensein des Enzyms anhand der Spaltung seiner Erkennungssequenz leicht nachgewiesen werden kann. Als Substrat eignet sich beispielsweise Lambda-DNA. Die erhaltenen DNA-Fragmente werden in Agarosegelen in den für die Fragmentauftrennung üblichen Puffersystemen, in Gegenwart von Ethidiumbromid, elektrophoretisch aufgetrennt.

Die für die Gewinnung des Enzyms verwendeten Mikroorganismen wachsen aerob in M 111-Medium (10g/l yeast extract + 10g/l Malzextrakt).
Die optimalen Wachstumsbedingungen sind bei 26 °C, pH 6,5 - 7,5. Die Verdoppelungszeit beträgt etwa 2,5 Stunden.

Das Enzym wird isoliert und gereinigt durch die üblichen chemischen und mechanischen Methoden, beispielsweise durch Hochdruckdispersion, Ultraschall oder enzymatischen Aufschluß. In einer bevorzugten Ausführungsform des erfindungsgemäßen Verfahrens werden die Zellen über eine French-Presse aufgeschlossen. Die weitere Reinigung des Überstands wird vorzugsweise über Affinitätschromatographie, und Ionentauscherchromatographie durchgeführt. Als Material für die Affinitätschromatographie geeignet ist beispielsweise Heparin-Sepharose CL-6B (Pharmacia). Ein geeigneter Kationenaustauscher ist beispielsweise Phospho-Cellulose (Whatman).

Als Anionentauscher geeignet ist das unter dem Namen DEAE Sephacel (Pharmacia) erhältliche Produkt. Aber auch andere Chromatographiematerialien, die dem Fachmann bekannt sind, sind geeignet.

Die folgenden Beispiele erläutern die Erfindung weiter.

Beispiel 1

Streptomyces griseus DSM 5621 wird bei 26°C 5 Stunden gezüchtet und in der logarithmischen Phase geerntet. Als Kulturmedium wird M 111-Medium verwendet.

Die Zellpaste (30 g Naßgewicht) wird in 2,4 Volumen Puffer A (40 mmol/l Tris-HCl, pH 8,0, 0,1 mmol/l EDTA, 7 mmol/l 2-Mercaptoethanol), welcher Proteaseinhibitoren enthält, resuspendiert. Anschließend werden die Zellen durch zweimalige Passage einer French-Presse bei 23.000 lb/inch$^2$ aufgeschlossen und der Niederschlag abgetrennt. Der Überstand wird mit NH$_4$Cl versetzt (Endkonzentration 0,3 mol/l) und mittels einer Polymin-Fällung die Nucleinsäuren entfernt. Anschließend wird der abzentrifugierte Überstand mit 55% Ammoniumsulfat behandelt und der Niederschlag über eine Heparin-Sepharose-Säule fraktioniert. Zur Elution wird ein Gradient von 0 - 1 mol/l NaCl verwendet. SgrAl wird in den Fraktionen zwischen 0,4 und 0,6 mol/l NaCl gefunden. Die aktiven Fraktionen werden gegen Puffer B (40 mmol/l Tris-HCl, pH 8,0; 0,1 mmol/l EDTA; 7 mmol/l 2-Mercaptoethanol; 10% (w/v) Glycerin) equilibriert und auf einer DEAE-Sephacel-Säule fraktioniert. Zur Elution wird ein Gradient von 0 - 0,5 mol/l NaCl verwendet. Die aktiven Fraktionen werden gegen Puffer B dialysiert.

Anschließend werden sie auf eine Phospho-Cellulose-Säule, welche mit Puffer B equilibriert wurde, aufgegeben. Zur Elution wird ein Gradient von 0 - 1 mol/l NaCl in Puffer B verwendet. SgrAl wird in den Fraktionen zwischen 0,3 und 0,5 mol/l NaCl gefunden.

Die aktiven Fraktionen werden zusammengegeben und gegen Lagerpuffer (20 mmol/l Tris-HCl, pH 8,0, 10 mmol/l 2-Mercaptoethanol, 100 mmol/l NaCl, 0,1 mmol/l EDTA und 50% (v/v) Glycerin) dialysiert.

Beispiel 2

Bestimmung der Aktivität

Definition der Enzymeinheiten: 1 U SgrAl spaltet 1 µg Lambda-DNA innerhalb 1 Stunde bei 37°C in 25 µl Endvolumen.

Zu einer Mischung von 2,5 µl Inkubationspuffer (100 mmol/l Tris-HCl, pH 7,5, 37°C, 100 mmol/l Magnesiumchlorid und 10 mmol/l DTE) werden 17,9 µl Wasser und 3,6 µl Lambda DNA (optische Dichte: 5,6 OD/ml) sowie 1 µl SgrAl-Lösung (1 U/µl) zugegeben. Die Lösung wird eine Stunde bei 37°C inkubiert, auf Eis gekühlt und mit 5 µl eines Abstopp-Reagenses, bestehend aus 7 mmol/l Harnstoff, 20 % (w/v) Saccharose, 60 mmol/l EDTA und 0,01 % (w/v) Bromphenolblau versetzt. Anschließend wird eine Trennung durch Elektrophorese in 1 % Agarose-Gelen für 3 - 4 Stunden bei 100 V durchgeführt. Die erhaltenen Banden werden über Vergleich mit einem DNA-Längenstandard identifiziert.

**Patentansprüche**

**1.** Typ II-Restriktionsendonuklease mit der Erkennungssequenz und der durch die Markierung charakterisierten Schnittstelle

$$5'-C\genfrac{}{}{0pt}{}{A}{G}\Big|CCGG\genfrac{}{}{0pt}{}{C}{T}G-3'$$
$$3'-G\genfrac{}{}{0pt}{}{C}{T}GGCC\Big|\genfrac{}{}{0pt}{}{A}{G}C-5',$$

erhältlich aus Mikroorganismen der Gattung Streptomyces.

**2.** Restriktionsendonuklease nach Anspruch 1, dadurch gekennzeichnet, daß sie aus Streptomyces griseus DSM 5621 erhältlich ist.

**3.** Restriktionsendonuklease nach den Ansprüchen 1 oder 2, gekennzeichnet durch ein Temperatur-Optimum bei ca. 37°C und ein pH-Optimum bei 8,0.

**4.** Verfahren zur Gewinnung einer TypII-Restriktionsendonuklease mit der Erkennungssequenz und der durch die Markierung charakterisierten Spaltstelle

$$5'\text{-C}^{\text{A}}_{\text{G}}|\text{CCGG}^{\text{C}}_{\text{T}}\text{G-3'}$$
$$3'\text{-G}^{\text{C}}_{\text{T}}\text{GGCC}^{\text{A}}_{\text{G}}|\text{C-5'},$$

dadurch gekennzeichnet, daß man Mikroorganismen der Gattung Streptomyces züchtet und das Enzym aus den Zellen gewinnt.

**5.** Verfahren nach Anspruch 4, dadurch gekennzeichnet, daß man Streptomyces griseus DSM 5621 züchtet.

**6.** Verfahren nach den Ansprüchen 4 und 5, dadurch gekennzeichnet, daß man die Zellen aufschließt und den Zellüberstand gewinnt.

**7.** Verfahren nach Anspruch 6, dadurch gekennzeichnet, daß man zur Feinreinigung den Zellüberstand einer Affinitätschromatographie, einer Anionenaustauschchromatographie und einer Kationenaustauschchromatographie unterwirft.

**8.** Verfahren nach Anspruch 7, dadurch gekennzeichnet, daß man zur Affinitätschromatographie trägerfixiertes Heparin verwendet.

**9.** Verwendung einer TypII-Restriktionsendonuklease mit der Erkennungssequenz und der durch die Markierung charakterisierten Schnittstelle

$$5'\text{-C}^{\text{A}}_{\text{G}}|\text{CCGG}^{\text{C}}_{\text{T}}\text{G-3'}$$
$$3'\text{-G}^{\text{C}}_{\text{T}}\text{CCGG}^{\text{A}}_{\text{G}}|\text{C-5'},$$

erhältlich aus Mikroorganismen der Gattung Streptomyces, zur Erkennung und Spaltung der komplementären doppelsträngigen DNA-Sequenz

$$5'\text{-C}^{\text{A}}_{\text{G}}\text{CCGG}^{\text{C}}_{\text{T}}\text{G-3'}$$

## Claims

**1.** Type II restriction endonuclease with the recognition sequence and the cleavage position characterised by the marking

$$5'\text{-C}^{\text{A}}_{\text{G}}|\text{CCGG}^{\text{C}}_{\text{T}}\text{G-3'}$$
$$3'\text{-G}^{\text{C}}_{\text{T}}\text{GGCC}|^{\text{A}}_{\text{G}}\text{C-5'}$$

obtainable from micro-organisms of the genus Streptomyces.

2. Restriction endonuclease according to claim 1, characterised in that it is obtainable from Streptomyces griseus DSM 5621.

3. Restriction endonuclease according to claim 1 or 2, characterised by a temperature optimum at about 37° C and a pH optimum at 8.0.

4. Process for the obtaining of a type II restriction endonuclease with the recognition sequence and the cleavage position characterised by the marking

$$5'-C{}^{A}_{G}\Big|CCGG{}^{C}_{T}G-3'$$
$$3'-G{}^{C}_{T}GGCC\Big|{}^{A}_{G}C-5'$$

characterised in that one cultures micro-organisms of the genus Streptomyces and obtains the enzyme from the cells.

5. Process according to claim 4, characterised in that one cultures Streptomyces griseus DSM 5621.

6. Process according to claims 4 and 5, characterised in that one digests the cells and recovers the cell supernatant.

7. Process according to claim 6, characterised in that, for the fine purification, one subjects the cell supernatant to an affinity chromatography, an anion exchange chromatography and a cation exchange chromatography.

8. Process according to claim 7, characterised in that one uses carrier-fixed heparin for the affinity chromatography.

9. Use of a type II restriction endonuclease with the recognition sequence and the cleavage position characterised by the marking

$$5'-C{}^{A}_{G}\Big|CCGG{}^{C}_{T}G-3'$$
$$3'-G{}^{C}_{T}CCGG\Big|{}^{A}_{G}C-5'$$

obtainable from micro-organisms of the genus Streptomyces for the recognition and cleavage of the complementary double-stranded DNA sequence

$$5'-C{}^{A}_{G}CCGG{}^{C}_{T}G-3'.$$

**Revendications**

1. Endonucléase de restriction de type II, reconnaissant la séquence ci-après, qu'elle coupe au site caractérisé par la marque

$$5'\text{-C}_{\,G}^{\,A}\big|\text{CCGG}_{\,T}^{\,C}\text{G-3'}$$
$$3'\text{-G}_{\,T}^{\,C}\text{GGCC}\big|_{\,G}^{\,A}\text{C-5'},$$

pouvant être obtenue à partir de microorganismes du genre Streptomyces.

2. Endonucléase de restriction selon la revendication 1, caractérisée en ce qu'elle peut être obtenue à partir de Streptomyces griseus DSM 5621.

3. Endonucléase de restriction selon la revendication 1 ou 2, caractérisée par une température optimale d'environ 37°C et un pH optimal de 8,0.

4. Procédé pour l'obtention d'une endonucléase de restriction de type II, reconnaissant la séquence ci-après, qu'elle coupe au site caractérisé par la marque

$$5'\text{-C}_{\,G}^{\,A}\big|\text{CCGG}_{\,T}^{\,C}\text{G-3'}$$
$$3'\text{-G}_{\,T}^{\,C}\text{GGCC}_{\,G}^{\,A}\text{C-5'},$$

caractérisé en ce que l'on cultive des microorganismes du genre Streptomyces et que l'on prélève l'enzyme à partir des cellules.

5. Procédé selon la revendication 4, caractérisé en ce que l'on cultive Streptomyces griseus DSM 5621.

6. Procédé selon les revendications 4 et 5, caractérisé par la rupture des cellules et la récupération du surnageant de l'extrait cellulaire.

7. Procédé selon la revendication 6, caractérisé en ce que, pour une purification fine, on soumet le surnageant cellulaire à une chromatographie d'affinité, une chromatographie échangeuse d'anions et une chromatographie échangeuse de cations.

8. Procédé selon la revendication 7, caractérisé en ce que pour la chromatographie d'affinité, on utilise de l'héparine fixée sur un support.

9. Utilisation d'une endonucléase de restriction de type II, reconnaissant la séquence ci-après, qu'elle coupe au site caractérisé par la marque

$$5'\text{-C}_{\,G}^{\,A}\big|\text{CCGG}_{\,T}^{\,C}\text{G-3'}$$
$$3'\text{-G}_{\,T}^{\,C}\text{CCGG}_{\,G}^{\,A}\text{C-5'},$$

pouvant être obtenue à partir de microorganismes du genre Streptomyces, pour la reconnnaissance et la coupure de la séquence complémentaire d'ADN double brin

$$5'\text{-}C_G^A\text{CCGG}_T^C G\text{-}3'\ .$$